# EUROPÄISCHE PATENTSCHRIFT

(11) **EP 1 658 107 B1**
(45) Veröffentlichungstag und Bekanntmachung des Hinweises auf die Patenterteilung: **27.12.2006**
(21) Anmeldenummer: 04764592.4
(22) Anmeldetag: 23.08.2004
(51) Int. Cl.: A61M 5/30, A61M 5/28, A61M 5/32

(54) **VORRICHTUNG ZUR INJEKTION EINES INJIZIERBAREN PRODUKTES**
DEVICE FOR INJECTING AN INJECTABLE PRODUCT
DISPOSITIF D'INJECTION D'UN PRODUIT INJECTABLE

(30) Priorität: 29.08.2003 DE 10340613
(43) Veröffentlichungstag der Anmeldung: 24.05.2006
(73) Patentinhaber: primoJEX GmbH, 16540 Hohen Neuendorf (DE)
(72) Erfinder: EICHHORST, Peter, 16540 Hohen-Neuendorf (DE); HEIDL, Wolfgang, 97702 Münnerstadt (DE)
(74) Vertreter: Schneider, Henry
(86) Internationale Anmeldenummer: PCT/EP2004/009618
(87) Internationale Veröffentlichungsnummer: WO 2005/023343

(56) Entgegenhaltungen:
- GB-A- 1 284 154
- US-A- 5 954 689
- US-A1- 2002 151 843
- US-B1- 6 217 550

## Beschreibung

Die Erfindung betrifft eine Vorrichtung zur Injektion eines injizierbaren Produktes, mit einem das Produkt aufnehmenden Vorratsbehälter, einer Austrittsanordnung zum Überführen des Produktes aus dem Vorratsbehälter und einer Betätigungsvorrichtung zur Beaufschlagung des Produktes in dem Vorratsbehälter mit einem Druck.

Vorrichtungen der gattungsgemäßen Art sind bekannt. Diese dienen der Injektion des injizierbaren Produktes, wobei unter injizierbarem Produkt insbesondere Flüssigkeiten, beispielsweise Lösungen, Suspensionen oder Dispersionen, die eine Wirksubstanz enthalten, verstanden werden. Wirksubstanzen können pharmakologisch aktive Stoffe zum Behandeln des menschlichen oder tierischen Körpers oder Stoffe zur Diagnose oder kosmetischen Anwendung sein. Ferner können die injizierbaren Produkte auch der Zuführung von Wirkstoffen in Pflanzen dienen.

Es sind Vorrichtungen der gattungsgemäßen Art zur subkutanen, intravenösen, intramuskulären oder intrakutanen Verabreichung der injizierbaren Produkte bekannt. Hierbei sind Vorrichtungen bekannt, bei denen die Verabreichung des injizierbaren Produktes durch eine Injektion unter Verwendung einer Kanüle erfolgt. Hierbei sind so genannte Einmalspritzen bekannt, die einen mit dem zu injizierenden Produkt vorfüllbaren Vorratsbehälter umfassen, der mit einer durch eine Schutzkappe abgedeckten Kanüle in Verbindung steht und dem andererseits eine Betätigungseinrichtung zugeordnet ist, mittels der ein Kolben innerhalb des Vorratsbehälters verlagerbar ist. Zum Gebrauch dieser Einmalspritze wird diese aus einer sterilen Blisterverpackung entnommen, die Schutzkappe von der Kanüle abgezogen und nach Setzen der Einmalspritze in dem zu beaufschlagenden Bereich des Körpers durch Druck auf die Betätigungseinrichtung das zu injizierende Produkt in das Gewebe überführt.

Andererseits sind so genannte nadellose Injektoren bekannt, mittels denen ein zu injizierendes Produkt unter Druck auf einen Gewebebereich aufgebracht wird, so dass dieses aufgrund des Druckes in das Gewebe eintritt. Derartige nadellose Injektoren besitzen einen Aufnahmebereich für eine vorgefüllte, das zu injizierende Produkt enthaltende Ampulle, eine Betätigungseinrichtung zum Beaufschlagen des Produktes mit einem Druck, so dass das Produkt durch eine Düsenanordnung austritt und in die gewünschten Gewebebereiche eindringt. Die Beaufschlagung mit dem Druck kann hier durch ein gegen die Kraft eines Federelementes vorspannbares Auslöseelement oder durch ein externes Druckmedium erfolgen.

Ob eine Einmalspritze oder ein nadelloser Injektor eingesetzt wird, richtet sich unter anderem nach der mit dem zu injizierenden Produkt beabsichtigten Wirkung. Je nachdem, ob das Produkt beispielsweise subkutan, intravenös oder intramuskulär verabreicht werden soll, steht entweder die Einmalspritze oder der nadellose Injektor als Injektionsvorrichtung zur Verfügung.

Der Erfindung liegt die Aufgabe zugrunde, eine Vorrichtung zur Injektion eines injizierbaren Produktes zu schaffen, die sich durch einen einfachen Aufbau auszeichnet und eine flexible Anwendung bei der Verabreichung injizierbarer Produkte ermöglicht.

Erfindungsgemäß wird diese Aufgabe durch eine Vorrichtung mit den im Anspruch 1 genannten Merkmalen gelöst. Dadurch, dass die Vorrichtung zur Injektion eines injizierbaren Produktes als Einmalspritze oder als Ampulle für einen nadellosen Injektor einsetzbar ist, ist vorteilhaft möglich, erst kurz vor Verabreichung des zu injizierenden Produktes zu entscheiden, ob dieses beispielsweise intramuskulär oder intravenös über die Einmalspritze oder beispielsweise subkutan über einen nadellosen Injektor verabreicht wird. Es ergibt sich somit die Möglichkeit, ein und dieselbe Vorrichtung wahlweise als Einmalspritze oder als Ampulle zu verwenden.

In bevorzugter Ausgestaltung der Erfindung ist vorgesehen, dass die Betätigungsvorrichtung einen Kolben umfasst, der dichtend in dem Vorratsbehälter geführt ist und der einen proximalen Bereich aufweist, der von einem distalen Bereich abtrennbar ist. Hierdurch wird in einfacher Weise möglich, eine Umrüstung der Betätigungsvorrichtung für den Fall vorzunehmen, wenn die Vorrichtung zur Injektion als Ampulle für einen nadellosen Injektor eingesetzt werden soll. Der proximale Bereich ist mit dem distalen Bereich des Kolbens vorzugsweise lösbar verbunden. Hierdurch kann durch Lösen dieser Verbindung, die beispielsweise eine Rastverbindung, ein Formschluss oder dergleichen sein kann, die Umrüstung mit einem einfachen Handgriff erfolgen. Ferner ist bevorzugt, wenn der proximale Bereich mit dem distalen Bereich über eine Sollbruchstelle verbunden ist. Hier kann durch einfaches Beaufschlagen des proximalen Bereiches mit einer Kraft der Kolben an der Sollbruchstelle abgebrochen werden, so dass ebenfalls eine einfache Umrüstung möglich ist.

Ferner ist in bevorzugter Ausgestaltung der Erfindung vorgesehen, dass die Austrittsanordnung zum Überführen des injizierbaren Produktes aus dem Vorratsbehälter eine Kanüle umfasst, deren distaler Bereich von einem proximalen Bereich der Kanüle definiert entfernbar ist. Hierdurch lässt sich in einfacher Weise die als Einmalspritze vorliegende Injektionsvorrichtung in eine Ampulle für einen nadellosen Injektor umrüsten. Die Kanüle weist hierzu vorzugsweise eine Sollbruchstelle auf, die bei Einsatz als Einmalspritze einen Durchgang bildet und bei Einsatz als Ampulle für einen nadellosen Injektor - bei entferntem distalen Ende der Kanüle - eine Austrittsdüse ausbildet.

Ferner ist in weiterer bevorzugten Ausgestaltung der Erfindung vorgesehen, dass der Austrittsanordnung eine Schutzkappe zugeordnet ist. Diese Schutzkappe dient in an sich bekannter Weise der schützenden Aufnahme der Kanüle, insbesondere von deren angeschliffenem Ende, und andererseits der Abdichtung des das vorgefüllte zu injizierende Produkt aufnehmenden Vorratsbehälters. Bevorzugt ist vorgesehen, dass die Schutzkappe eine Sollbruchstelle aufweist, entlang der ein distales Ende der Schutzkappe entfernbar ist, während ein proximales Ende der Schutzkappe am Vorratsbehälter verbleibt. Hierdurch wird erreicht, dass das am Vorratsbehälter verbleibende proximale Ende der Schutzkappe eine Schutzfunktion des Vorratsbehälters, insbesondere bei dessen Einsatz als vorgefüllte Ampulle in einem nadellosen Injektor übernimmt. Das verbleibende proximale Ende bildet einerseits eine selbstjustierende Führung für den Vorratsbehälter in einem nadellosen Injektor und andererseits einen Ringanker im Düsenbereich des Vorratsbehälters. Hierdurch wird vorteilhaft erreicht, dass ein Zerbersten des Vorratsbehälters bei hohen, bei Anwendung als nadellosen Injektor auftretenden Drücken verhindert wird.

In weiterer bevorzugter Ausgestaltung der Erfindung ist vorgesehen, dass die Vorrichtung für eine Langzeitlagerung der zu injizierenden Produkte, insbesondere von Medikamenten, geeignet ist. Hier ist insbesondere vorgesehen, dass der Vorratsbehälter aus Glas, insbesondere Borosilikatglas, zumindest die mit dem Produkt in Berührung kommenden Teile der Schutzkappe und der Betätigungsvorrichtung aus Butylgummi und die Kanüle aus chirurgischem Edelstahl besteht.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figuren 1 bis 3: verschiedene Ansichten einer als Einmalspritze eingesetzten Vorrichtung zur Injektion eines injizierbaren Produktes und
- Figuren 4 bis 10: verschiedene Ansichten einer als Ampulle für eine als nadelloser Injektor eingesetzte Vorrichtung zur Injektion eines injizierbaren Produktes.

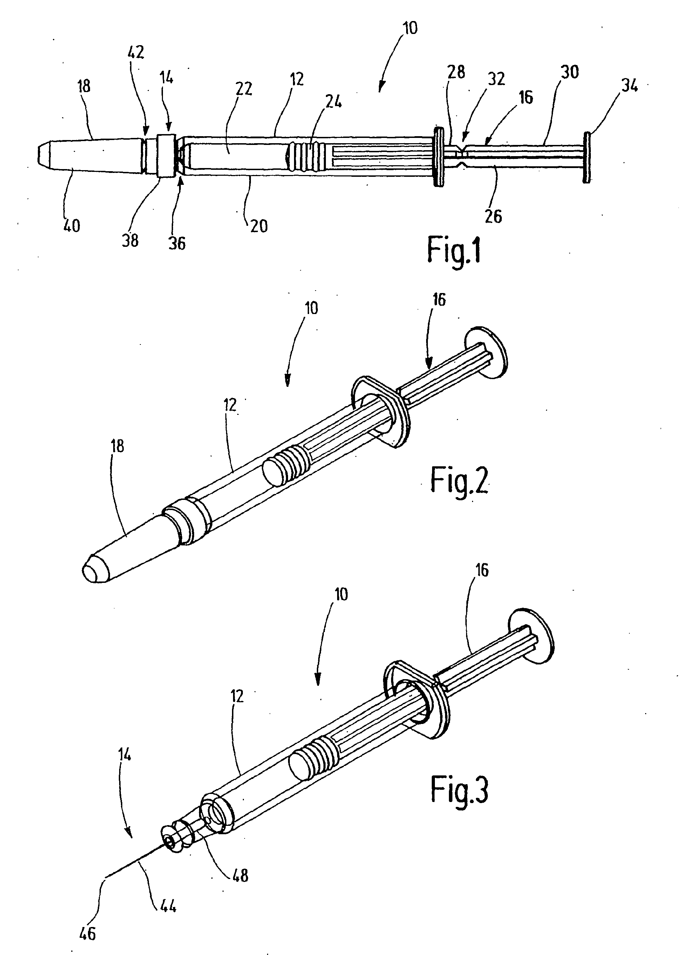

Figur 1 zeigt eine insgesamt mit 10 bezeichnete Vorrichtung zur Injektion eines injizierbaren Produktes. Die Vorrichtung 10 umfasst einen Vorratsbehälter 12, eine Austrittsanordnung 14 sowie eine Betätigungsvorrichtung 16. In der in Figur 1 gezeigten Darstellung ist die Austrittsanordnung 14 durch eine Schutzkappe 18 abgedeckt. Der Vorratsbehälter 12 wird von einem zylinderförmigen Hohlkörper 20 gebildet, der einen Innenraum 22 besitzt. Der Hohlkörper 20 besteht beispielsweise aus Glas, insbesondere Borosilikatglas, kann jedoch auch aus anderen geeigneten Materialien, beispielsweise Kunststoff, Metall oder dergleichen, bestehen.

Der Innenraum 22 wird einerseits - am proximalen Ende - von einem Pfropfen 24 begrenzt, der mit einer Kolbenstange 26 in Verbindung steht. Der Pfropfen 24 besteht beispielsweise aus Butylgummi, während die Kolbenstange 26 beispielsweise aus einem Kunststoff besteht. Die Kolbenstange 26 besitzt einen distalen Bereich 28 und einen proximalen Bereich 30, die über eine Sollbruchstelle 32 miteinander verbunden sind. Der proximale Bereich 30 wird durch eine Daumendruckplatte 34 abgeschlossen.

Die Schutzkappe 18 ist im Wesentlichen rotationssymmetrisch ausgebildet und auf ein distales Ende 36 des Vorratsbehälters 12 aufgesteckt, aufgerastet oder dergleichen. Hierzu besitzt ein proximaler Endabschnitt 38 der Schutzkappe 18 und das distale Ende 36 des Vorratsbehälters 12 entsprechende korrespondierende Formmerkmale. Ein distales Ende 40 der Schutzkappe 18 ist mit dem proximalen Ende 38 über eine Sollbruchstelle 42 verbunden. Die Schutzkappe 18 besteht beispielsweise aus Butylgummi.

Figur 2 zeigt die Vorrichtung 10 in einer Perspektivansicht, wobei gleiche Teile wie in Figur 1 mit gleichen Bezugszeichen versehen und nicht nochmals erläutert sind.

Bei der in Figur 3 dargestellten Perspektivansicht ist die Schutzkappe 18 abgenommen, so dass die Austrittsanordnung 14 freigelegt ist. Die Austrittsanordnung 14 umfasst eine Kanüle 44, deren distales Ende einen Anschliff 46 besitzt und deren proximales Ende in eine distale Öffnung 48 des Vorratsbehälters 12 eingebracht ist. Die Kanüle 44 ist beispielsweise mittels eines UV-härtbaren Klebers in der Öffnung 48 eingeklebt. Die Kanüle 44 besteht beispielsweise aus chirurgischem Edelstahl.

Die in den Figuren 1 bis 3 dargestellte Vorrichtung 10 zeigt folgende Funktion:

Der Vorratsbehälter 12 wird in an sich bekannter Weise mittels einer Ready-to-fill-Abfüllanlage mit dem zu injizierenden Produkt, beispielsweise einen pharmakologischen Wirkstoff enthaltende Lösung, Dispersion, Suspension oder dergleichen, befüllt und mit dem Pfropfen 24 und der Betätigungsvorrichtung 16 verschlossen und komplettiert. Der Innenraum 22 enthält somit eine definierte Menge eines zu injizierenden Produktes. Die Kanüle 44 ist über die Schutzkappe 18 geschützt, wobei gleichzeitig ein Austritt des Produktes aus dem Innenraum 22 verhindert wird. Die Vorrichtung 10 wird anschließend in ebenfalls bekannter Weise in einer Blisterverpackung verpackt. Durch Wahl der Materialien und Verpackung der Vorrichtung 10 ist eine Langzeitlagerung möglich.

Entsprechend den Darstellungen in den Figuren 2 und 3 wird die Vorrichtung 10 als so genannte Einmalspritze, beispielsweise zum intravenösen, intramuskulären Verabreichen des zu injizierenden Produktes verwendet. Hierzu wird die Schutzkappe 18 vom Vorratsbehälters 12 abgezogen und mittels der Betätigungsvorrichtung 16 das Produkt wunschgemäß verabreicht.

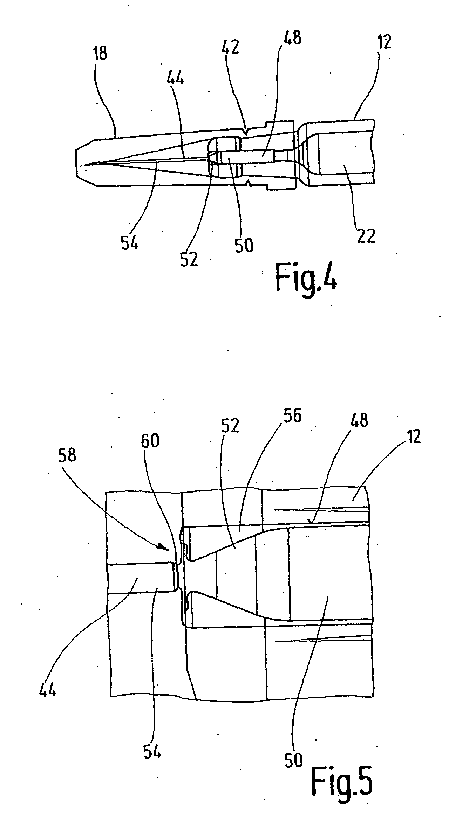

Anhand der nachfolgenden Figuren 4 bis 10 wird auf den Einsatz der in Figur 1 dargestellten Vorrichtung 10 als Ampulle für einen nadellosen Injektor eingegangen. Wie bereits erläutert, kann die Vorrichtung 10 entsprechend der Darstellung in Figur 3 auch als Einmalspritze eingesetzt werden.

Figur 4 zeigt eine schematische Schnittansicht der Vorrichtung 10 im Bereich von deren distalem Ende, wobei gleiche Teile wie in den vorhergehenden Figuren mit gleichen Bezugszeichen versehen sind. Es wird deutlich, dass die Schutzkappe 18 die Kanüle 44 umschließt, die in der distalen Öffnung 48 des Vorratsbehälters 12 eingeklebt ist. Die Kanüle 44 besitzt einen innerhalb der Öffnung 48 liegenden Abschnitt. 50, der über einen konisch verlaufenden Abschnitt 52 in die eigentliche Kanüle 44 übergeht. Der Abschnitt 50 besitzt hierbei einen größeren Außendurchmesser als der Abschnitt 54 der Kanüle 44. Der Abschnitt 50 besitzt beispielsweise einen Außendurchmesser von 1,2 mm und einen Innendurchmesser von 0,8 mm, während der Abschnitt 54 beispielsweise einen Außendurchmesser von 0,4 mm und einen Innendurchmesser von 0,2 mm besitzt. Die Anpassung der Außendurchmesser beziehungsweise Innendurchmesser der Abschnitte 50 und 54 erfolgt über den konischen Abschnitt 52. In einer weiteren Detailvergrößerung in Figur 5 wird deutlich; dass die Kanüle 44 über ihre Abschnitte 50 und 52 mittels eines UV-härtbaren Klebers 56 in der Öffnung 48 des Vorratsbehälters 12 fixiert ist. Ferner wird deutlich, dass im Übergangsbereich zwischen dem konischen Bereich 52 und dem Abschnitt 54 der Kanüle 44 eine Sollbruchstelle 58 vorgesehen ist. Diese ist als randoffene Ringnut 60 in die Kanüle 44 eingearbeitet.

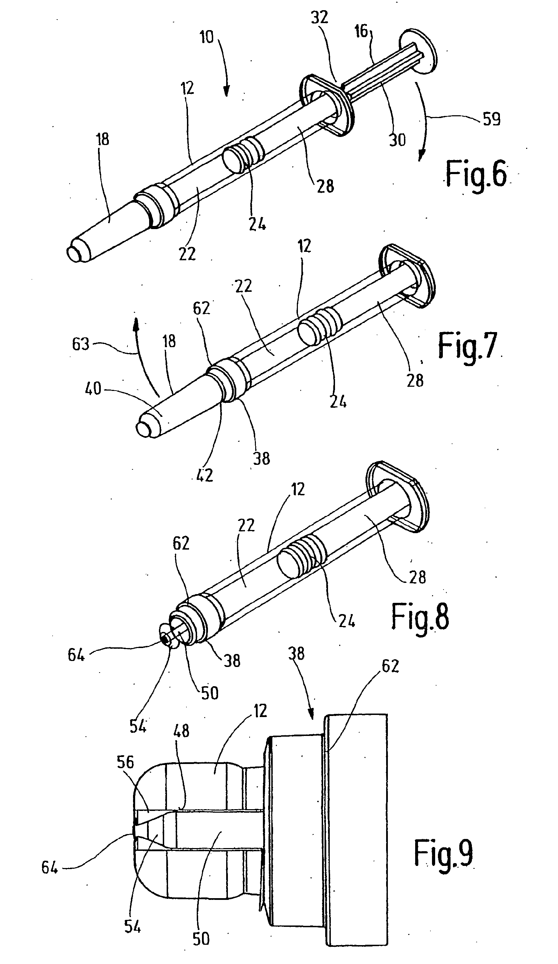

Anhand der Figuren 6 bis 10 wird der Einsatz der in den Figuren 1 bis 5 beschriebenen Vorrichtung 10 als Ampulle für einen nadellosen Injektor beschrieben. Als Ausgangssituation ergibt sich die in Figur 6 (entspricht der Darstellung in Figur 2 für den Einsatz als Einmalspritze) gezeigte grundsätzliche Anordnung. In einem ersten Schritt wird, wie Figur 7 verdeutlicht, der proximale Bereich 30 der Kolbenstange 26 an der Sollbruchstelle 32 abgebrochen (Pfeil 59 in Figur 6). Dies kann durch einfaches Umknicken des proximalen Bereiches 30 relativ zum innerhalb des Vorratsbehälters 12 angeordneten distalen Bereich 28 der Kolbenstange 26 erfolgen. Es ergibt sich somit die in Figur 7 gezeigte Anordnung. Die Anordnung gemäß Figur 7 wird in den Aufnahmebereich eines nicht dargestellten nadellosen Injektors eingelegt. Hierbei dient die Schutzkappe 18 als Justagehilfe, indem der Vorratsbehälter 12 mit der Schutzkappe 18 in den entsprechenden Aufnahmebereich eingeschoben wird, wobei ein Bund 62 als Positionierungsanschlag dient.

Anschließend wird, wie Figur 8 verdeutlicht, dass distale Ende 40 der Schutzkappe 18 relativ zum nadellosen Injektor und somit zum innerhalb des nadellosen Injektors angeordneten Vorratsbehälter 12 umgebogen (Pfeil 63 in Figur 7). Die relativ elastische Schutzkappe 18 folgt dieser Bewegung. Durch diese Umknickbewegung wird die Kanüle 44 an ihrer Sollbruchstelle 58 (Figur 5) abgebrochen. Anschließend wird das distale Ende 40 der Schutzkappe 18 an der Sollbruchstelle 42 abgerissen, so dass die in Figur 8 gezeigte Anordnung innerhalb des nadellosen Injektors verbleibt. Das proximale Ende 38 der Schutzkappe 18 dient weiterhin der Lagepositionierung des Vorratsbehälters 12 innerhalb des nadellosen Injektors. Andererseits bildet das proximale Ende 38 der Schutzkappe 18 einen das distale Ende des Vorratsbehälters 12 umgreifenden Ringanker. In der vergrößerten Darstellung in Figur 9 wird dies nochmals deutlich. Die sich an der Sollbruchstelle 58 der Kanüle 44 ergebende Öffnung 64 bildet die Düsenöffnung zum Austritt des zu injizierenden Produktes. Der Auslösemechanismus des nicht dargestellten nadellosen Injektors wird auf das distale Ende 36 der Kolbenstange 26 beschleunigt, so dass das im Innenraum 22 vorhandene, zu injizierende Produkt über den Pfropfen 24 mit einem Druck beaufschlagt wird. Entsprechend dem plötzlich auftretenden hohen Druck wird das Produkt über den in der Öffnung 48 verbliebenen proximalen Abschnitt 50 der Kanüle und den konischen Abschnitt 54 zur Düsenöffnung 64 gedrängt, so dass das Produkt mit dem erforderlichen Druck austritt und beispielsweise subkutan verabreicht werden kann.

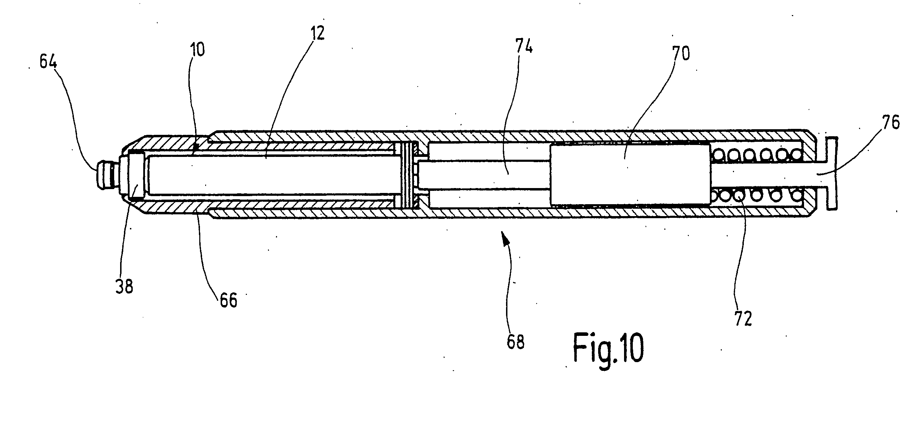

Figur 10 zeigt schließlich nochmals eine Gesamtanordnung, bei der die Vorrichtung 10 in einen nadellosen Injektor eingelegt ist. Die entsprechend der in den Figuren 6 bis 9 veränderte Vorrichtung 10 dient nunmehr als vorgefüllte Ampulle für den Injektor. Die Ampullen können dabei in Einweg- oder Mehrfachinjektoren eingesetzt werden.

Anhand der vorstehenden Erläuterungen wird deutlich, dass die Vorrichtung 10, wie diese in Figur 1 gezeigt ist, sowohl als Einmalspritze (Figur 3) als auch als Ampulle für einen nadellosen Injektor (Figur 10) einsetzbar ist. Mit wenigen Handgriffen ist die Vorrichtung 10 für den nadellosen Injektor umrüstbar oder eben ohne Umrüstung als Einmalspritze einsetzbar. Somit ergeben sich vielfältige flexible Anwendungsmöglichkeiten der Vorrichtung 10 je nach gewünschter Anwendung. Das separate Herstellen und Vorrätighalten von Einmalspritzen einerseits und Ampullen für nadellose Injektoren andererseits ist somit nicht erforderlich. Insbesondere ergeben sich auch hinsichtlich der identischen Abmaße der Vorratsbehälter 12 wesentliche Vorteile beim Abfüllen, da ein Umrüsten vorhandener Ready-to-fill-Abfüllanlagen nicht erforderlich ist.

### Bezugszeichenliste

- 10: Vorrichtung
- 12: Vorratsbehälter
- 14: Austrittsanordnung
- 16: Betätigungsvorrichtung
- 18: Schutzkappe
- 20: Hohlkörper
- 22: Innenraum
- 24: Pfropfen
- 26: Kolbenstange
- 28: distaler Bereich
- 30: proximaler Bereich
- 32: Sollbruchstelle
- 34: Daumendruckplatte
- 36: distales Ende
- 38: proximales Ende
- 40: distales Ende
- 42: Sollbruchstelle
- 44: Kanüle
- 46: Anschliff
- 48: distale Öffnung
- 50: zylindrischer Abschnitt
- 52: konischer Abschnitt
- 54: Abschnitt
- 56: UV-härtbarer Kleber
- 58: Sollbruchstelle
- 59: Abbruchvorgang
- 60: randoffene Ringnut
- 62: Bund
- 63: Umbiegevorgang
- 64: Austrittsdüse

## Patentansprüche

1. Vorrichtung zur Injektion eines injizierbaren Produktes, mit einem das Produkt aufnehmenden Vorratsbehälter (12), einer Austrittsanordnung (14) zum Überführen des Produktes aus dem Vorratsbehälter und einer Betätigungsvorrichtung (16) zur Beaufschlagung des Produktes in dem Vorratsbehälter (12) mit einem Druck, **dadurch gekennzeichnet, dass** die Vorrichtung (10) wahlweise als Einmalspritze verwendbar oder als Ampulle für einen nadellosen Injektor einsetzbar ist.

2. Vorrichtung nach Anspruch 1, **dadurch gekennzeichnet, dass** die Betätigungsvorrichtung (16) eine Kolbenstange (26) umfasst, die einen proximalen Bereich (30) aufweist, der von einem distalen Bereich (28) abtrennbar ist.

3. Vorrichtung nach Anspruch 2, **dadurch gekennzeichnet, dass** der proximale Bereich (30) mit dem distalen Bereich (28) lösbar verbunden ist.

4. Vorrichtung nach Anspruch 2, **dadurch gekennzeichnet, dass** der proximale Bereich (30) mit dem distalen Bereich (28) über eine Sollbruchstelle (32) verbunden ist.

5. Vorrichtung nach einem der vorhergehenden Ansprüche, **dadurch gekennzeichnet, dass** die Austrittsanordnung (14) eine Kanüle (44) umfasst, deren distaler Bereich von einem proximalen Bereich der Kanüle (44) über eine Sollbruchstelle (58) definiert entfernbar ist.

6. Vorrichtung nach Anspruch 5, **dadurch gekennzeichnet, dass** der proximale Bereich der Kanüle (44) bei Einsatz als Ampulle für einen nadellosen Injektor eine Austrittsdüse (64) für das Produkt ausbildet.

7. Vorrichtung nach einem der vorhergehenden Ansprüche, **dadurch gekennzeichnet, dass** der proximale Bereich der Kanüle (44) einen größeren Außendurchmesser und Innendurchmesser als der distale Bereich der Kanüle aufweist.

8. Vorrichtung nach Anspruch 7, **dadurch gekennzeichnet, dass** der proximale Bereich einen zylindrischen Abschnitt (50) und einen konischen Abschnitt (52) aufweist, der in die Austrittsdüse (64) übergeht.

9. Vorrichtung nach einem der vorhergehenden Ansprüche, **dadurch gekennzeichnet, dass** der Austrittsanordnung (14) eine Schutzkappe (18) zugeordnet ist.

10. Vorrichtung nach Anspruch 9, **dadurch gekennzeichnet, dass** die Schutzkappe (18) eine Sollbruchstelle (42) aufweist, entlang der ein distales Ende (40) der Schutzkappe (18) entfernbar ist.

11. Vorrichtung nach einem der Ansprüche 9 und 10, **dadurch gekennzeichnet, dass** ein proximales Ende (38) der Schutzkappe (18) einen Ringanker für den Vorratsbehälter (12) bildet.

12. Vorrichtung nach einem der Ansprüche 9 bis 11, **dadurch gekennzeichnet, dass** das proximale Ende (38) eine selbstjustierende Führung für die Vorrichtung (10) in einen nadellosen Injektor bildet.

13. Vorrichtung nach einem der vorhergehenden Ansprüche, **dadurch gekennzeichnet, dass** die Vorrichtung (10) aus für eine Langzeitlagerung der Produkte geeigneten Materialien, insbesondere Glas, Borosilikatglas, Butylgummi, chirurgischer Edelstahl, besteht.

## Claims

1. Device for injection of an injectable product, with a storage receptacle (12) that holds the product, a withdrawal arrangement (14) for transferring the product out of the storage receptacle, and an actuation device (16) for applying pressure to the product in the storage receptacle (12), **characterized in that** the device (10) can be used selectively as a disposable syringe or as an ampoule for a needle-free injector.

2. Device according to claim 1, **characterized in that** the actuating device (16) comprises a plunger rod (26) that has a proximal area (30) that can be separated from a distal area (28).

3. Device according to claim 2, **characterized in that** the proximal area (30) is detachably connected to the distal area (28).

4. Device according to claim 2, **characterized in that** the proximal area (30) is connected to the distal area (28) via a scored site (32).

5. Device according to one of the preceding claims, **characterized in that** the withdrawal arrangement (14) comprises a needle (44) whose distal area can be removed in a defined manner from a proximal area of the needle (44) by way of a scored site (58).

6. Device according to claim 5, **characterized in that** the proximal area of the needle (44) in an application as an ampoule for a needle-free injector forms a discharge nozzle (64) for the product.

7. Device according to one of the preceding claims, **characterized in that** the proximal area of the needle (44) has a larger outside diameter and inside diameter than the distal area of the needle.

8. Device according to claim 7, **characterized in that** the proximal area has a cylindrical section (50) and a conical section (52) that passes into the discharge nozzle (64).

9. Device according to one of the preceding claims, **characterized in that** a protective cap (18) is assigned to the withdrawal arrangement (14).

10. Device according to claim 9, **characterized in that** the protective cap (18) has a scored site (42) along which a distal end (40) of the protective cap (18) can be removed.

11. Device according to one of claims 9 and 10, **characterized in that** a proximal end (38) of the protective cap (18) forms a ring anchor for the storage receptacle (12).

12. Device according to one of claims 9 to 11, **characterized in that** the proximal end (38) forms a self-adjusting guide for the device (10) into a needle-free injector.

13. Device according to one of the preceding claims, **characterized in that** the device (10) consists of materials that are suited for long-term storage of products, especially glass, borosilicate glass, butyl rubber, and high-grade surgical steel.

## Revendications

1. Dispositif pour l'injection d'un produit injectable, avec un réservoir (12) contenant le produit (12), un dispositif d'écoulement (14) pour l'éjection du produit hors du réservoir et un dispositif de manipulation (16) pour l'application d'une pression sur le produit dans le réservoir (12), **caractérisé en ce que** le dispositif (10) est utilisable au choix comme seringue à usage unique ou comme ampoule pour un injecteur sans aiguille.

2. Dispositif selon la revendication 1, **caractérisé en ce que** le dispositif de manipulation (16) comprend une tige de piston (26) présentant une zone proximale (30) séparable d'une zone distale (28).

3. Dispositif selon la revendication 2, **caractérisé en ce que** la zone proximale (30) est raccordée à la zone distale (28) de manière à en être amovible.

4. Dispositif selon la revendication 2, **caractérisé en ce que** la zone proximale (30) est raccordée par un emplacement de rupture obligé (32) à la zone distale (28).

5. Dispositif selon l'une des revendications précédentes, **caractérisé en ce que** le dispositif d'écoulement (14) comporte une canule (44) dont la zone distale est retirable d'une zone proximale de la canule (44), d'une manière définie par un emplacement de rupture obligé (58).

6. Dispositif selon la revendication 5, **caractérisé en ce que** la zone proximale de la canule (44) forme une buse d'écoulement (64) pour le produit en cas d'utilisation comme ampoule pour un injecteur sans aiguille.

7. Dispositif selon l'une des revendications précédentes, **caractérisé en ce que** la zone proximale de la canule (44) présente un diamètre extérieur et un diamètre intérieur supérieurs à la zone distale de la canule.

8. Dispositif selon la revendication 7, **caractérisé en ce que** la zone proximale présente une partie cylindrique (50) et une partie conique (52), laquelle est terminée par la buse d'écoulement (64).

9. Dispositif selon l'une des revendications précédentes, **caractérisé en ce que** le dispositif d'écoulement (14) est doté d'un capuchon de protection (18).

10. Dispositif selon la revendication 9, **caractérisé en ce que** le capuchon de protection (18) présente un emplacement de rupture obligé (42), le long duquel une extrémité distale (40) du capuchon de protection (18) est retirable.

11. Dispositif selon l'une des revendications 9 et 10, **caractérisé en ce qu'**une extrémité proximale (38) du capuchon de protection (18) forme un ancrage annulaire pour le réservoir (12).

12. Dispositif selon l'une des revendications 9 à 11, **caractérisé en ce que** l'extrémité proximale (38) forme un guidage auto-ajustant pour le dispositif (10) dans un injecteur sans aiguille.

13. Dispositif selon l'une des revendications précédentes, **caractérisé en ce que** le dispositif (10) est réalisé dans des matériaux adaptés pour un stockage de longue durée des produits, en particulier du verre, du verre en silicate de bore, du caoutchouc butyle, de l'acier spécial de qualité chirurgicale.
